(19) Europäisches Patentamt
European Patent Office
Office européen des brevets

(11) Publication number: **0 450 492 A1**

(12) **EUROPEAN PATENT APPLICATION**

(21) Application number: **91104876.7**

(22) Date of filing: **27.03.91**

(51) Int. Cl.5: **C07C 329/16**, C08F 2/48, C08F 36/18, C08F 136/18

(30) Priority: **06.04.90 JP 90122/90**
**10.04.90 JP 93057/90**

(43) Date of publication of application:
**09.10.91 Bulletin 91/41**

(84) Designated Contracting States:
**DE FR GB IT**

(71) Applicant: **Tosoh Corporation**
**4560, Kaisei-cho**
**Shinnanyo-shi, Yamaguchi-ken 746(JP)**

(72) Inventor: **Yamakawa, Hiroshi**
**1401-21, Nakajima-cho**
**Kudamatsu-shi, Yamaguchi-ken(JP)**
Inventor: **Ozoe, Shinji**
**2-3-35, Miyanomae, Oaza-Tonda**
**Shinnanyo-shi, Yamaguchi-ken(JP)**

(74) Representative: **Vossius & Partner**
**Siebertstrasse 4 P.O. Box 86 07 67**
**W-8000 München 86(DE)**

(54) **Alkylxanthate, production thereof, photopolymerization initiator, and polymerization employing the same.**

(57) Alkylxanthate compound having the general formula:

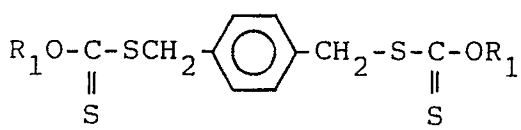

where $R_1$ is an alkyl group of 1 to 18 carbons. The compound is useful as an initiator of photopolymerization of various radical-polymerizable monomers, and produced by reacting an aliphatic alcohol having the general formula:

$R_2OH$

where $R_2$ is an alkyl group of 1 to 18 carbons with carbon disulfide, alkali metal hydroxide or alkali metal, and p-xylylene dichloride. Photopolymerization initiator comprising the alkylxanthate. Process for producing a polymer having a xanthate end group at the both ends, comprising irradiating ultraviolet ray to a mixture of the alkylxanthate and a radical-polymerizable monomer and chloroprene polymer having the general formula:

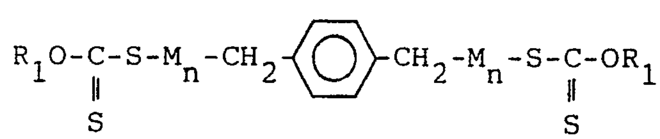

where $R_1$ is an alkyl group of 1 to 18 carbons; M is a chloroprene radical; n denotes the polymerization degree, and having a number-average molecular weight of from 1,000 to 15,000.

EP 0 450 492 A1

The present invention relates to a novel alkylxanthate and a process for producing the alkylxanthate. The compound of the present invention is useful as an initiator of photopolymerization of various radical-polymerizable monomers including diene monomers such as chloroprene, and acrylate monomers.
Further, the resulting polymer is useful as a polymeric photopolymerization initiator and as an molded article in a wide range of applications such as hoses, wires, and cable coating.

Heretofore, in photopolymerization of a radical polymerizable monomer by ultraviolet ray, there have been used disulfide compounds such as tetraalkylthiuram disulfide, tetraalkylxanthogen disulfide, and xylylene bis(dialkylcarbamate).
xylylene bis(dialkylcarbamate) is capable of inducing photopolymerization of variety of monomers such as diene monomers, styrene or styrene derivatives, acrylic acid and acrylate monomers like acrylic esters.
Disulfide compounds, however, are less active in initiation of photopolymerization, and serve as a chain-transfer agent. Therefore, the disulfide compounds are usually used combinedly with a radical polymerization initiator. Many papers have been published regarding photopolymerization: e.g., Ohtsu: J. Macromol. Sci. Chem. Vol. A21, No. 8 & 9, p. 961 (1984). The disulfide type photopolymerization initiators are disadvantageous in that the polymerization-initiating activity and photolysis ability are low and the polymerization therewith will not proceed quantitatively, and further with combined use of a radical polymerization initiator, the molecular weight of the polymer cannot readily be controlled. On the other hand, the carbamate type of photopolymerization initiators are disadvantageous in that the polymers prepared therewith are colored dark yellow to brown. In particular, when the above-mentioned carbamate type initiator is used, the initiator may not be consumed completely depending on the kind of monomer, making the molecular-weight control infeasible, which is a serious disadvantage. This tendency is remarkable in preparation of a low molecular polymer where a large amount of initiator is employed, thus the control of molecular weight being difficult in a wide range of from a low molecular weight to a high molecular weight. Additionally, no report has been found regarding the successful molecular weight control by use of a conventional carbamate type compound in polymerization of a diene monomer such as chloroprene.

As described above, the carbamate compound conventionally employed for photopolymerization makes it difficult and disadvantageous to control the molecular weight of the polymer in a wide range, and causes coloration of the polymer.

It is the object of the present invention to provide alkylxanthates which enable control of the molecular weight of a photopolymerized polymer in a wide range without coloration of the polymer, which has not been achieved by the carbamate compounds conventionally employed in photopolymerization.

According to an aspect of the present invention, there is provided an alkylxanthate compound represented by the general formula (I):

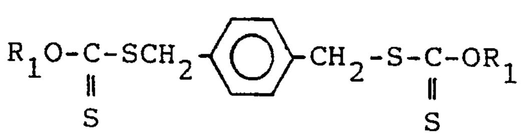

(I)

where $R_1$ is an alkyl group of 1 to 18 carbons.

According to another aspect of the present invention, there is provided a process for producing the compound of the formula (I), comprising reacting one mole of an aliphatic alcohol represented by the general formula (II)

$R_2OH$ (II)

(where $R_2$ is an alkyl group of 1 to 18 carbons) with one mole of carbon disulfide, one mole of alkali metal hydroxide or alkali metal, and 0.5 mole of p-xylylene dichloride.

According to still another aspect of the present invention, there is provided a photopolymerization initiator comprising the alkylxanthate represented by the general formula (I) above.

According to a further aspect of the present invention, there is provided a process for producing a polymer having a xanthate end group at both ends, comprising irradiating a mixture of an alkylxanthate represented by the general formula (I) above and a radical-polymerizable monomer with ultraviolet light to cause polymerization.

According to a still further aspect of the present invention, there is provided a chloroprene polymer represented by the general formula (III):

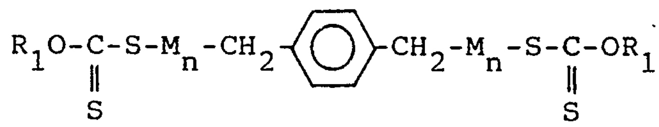

(III)

where $R_1$ is an alkyl group of 1 to 18 carbons; M is a chloroprene radical; n denotes the polymerization degree, and having a number-average molecular weight of from 1,000 to 15,000.

Fig. 1 shows GPC elution curves of the polymer derived in Example 5 (Curve 1), Example 6 (Curve 2), Comparative Example 1 (Curve 3), and Comparative Example 2 (Curve 4).

Fig. 2 shows $^1H$-NMR pattern of the polymer derived in Example 6.

As the result of comprehensive investigation on polymerization activity of various compounds to offset the above-described disadvantages of prior art, it has been found that polymerization of a diene monomer or acrylic ester monomer by use of a xylylene bis(alkylxanthate) represented by the general formula (I) above as a photopolymerization initiator gives a polymer without coloration thereof at an improved yield, in comparison with a carbamate initiator, and even at the charging ratio (molar ratio) of monomer/initiator of as low as not more than 50, and that the initiator does not remain unreacted, enabling satisfactory control of the molecular weight.

The xanthate of the present invention represented by the general formula (I):

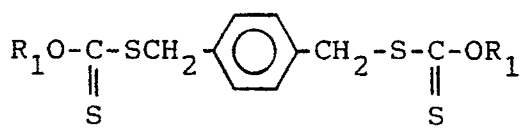

(I)

where $R_1$ is an alkyl group of 1 to 18 carbons, includes various compounds. The specific examples are as below:

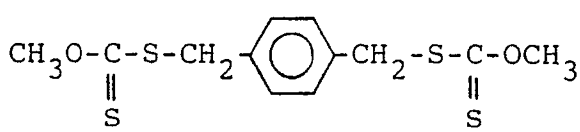

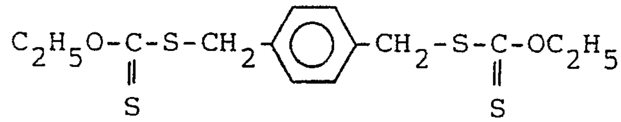

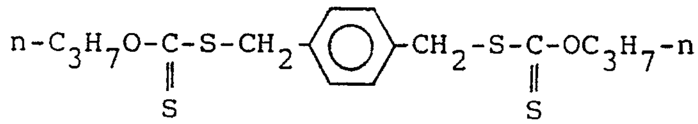

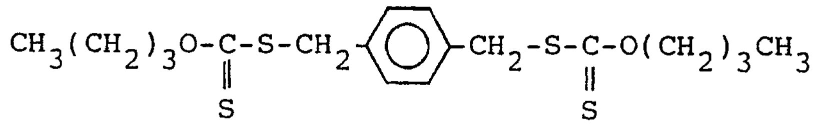

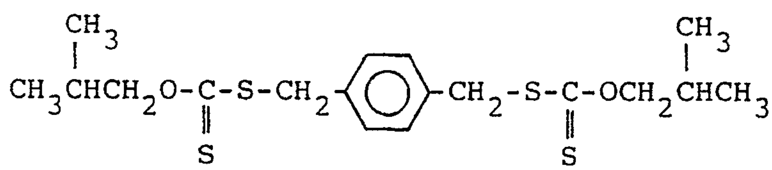

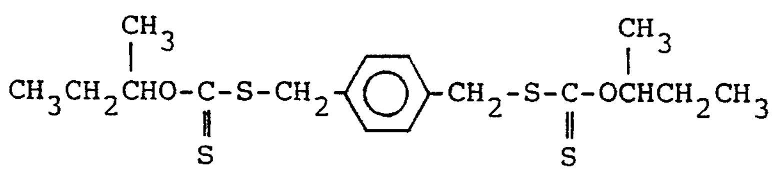

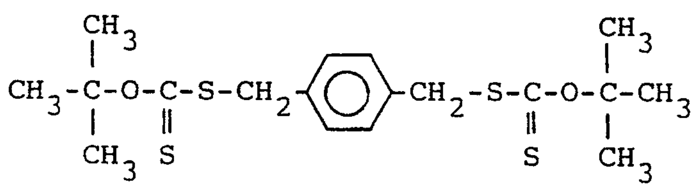

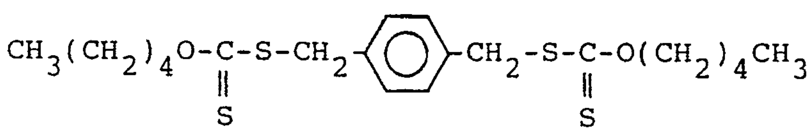

The above-described compounds are readily produced according to a method shown by the formulas (IV).

$$C_nH_{2n+1}\text{-}OH + KOH \rightarrow C_nH_{2n+1}\text{-}OK$$

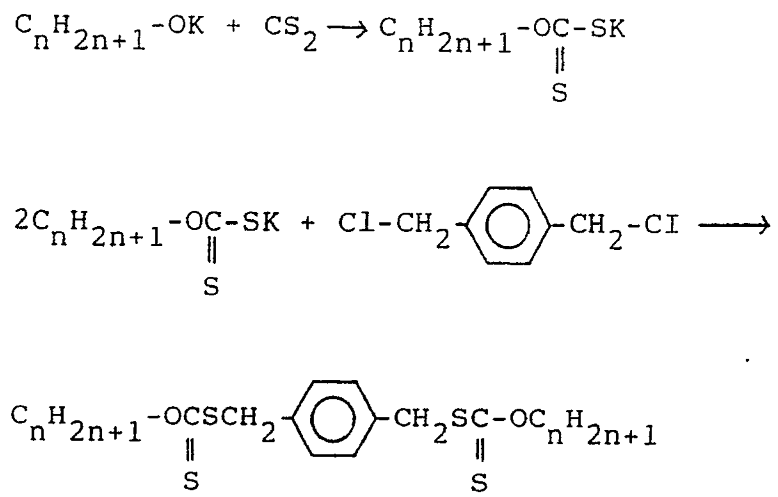

(IV)

The alcohols which can be employed for producing the xanthates of the present invention include e.g. methanol, ethanol, n-propanol, isopropanol, n-butanol, t-butanol, sec-butanol, n-pentanol and n-hexanol. Alcohols of 7 or more carbons can also be employed similarly.

The alkaline compounds include alkali metal hydroxides such as sodium hydroxide and potassium hydroxide and alkali metals such as sodium, lithium and potassium.

The p-xylylene dichloride is preferably recrystallized before use. On the other hand, the alcohols and the carbon disulfide need not be purified specially for the reaction.

The reaction solvent for production of the xanthate is preferably an alcohol which is the same as the alcohol of the starting material of the xanthate. However, non-halogen type aromatic hydrocarbons may also be usable.

The reaction temperature is preferably in the range of from 0°C to 50°C, more preferably from 0°C to 30°C, although the temperature is not specially limited thereto.

The process for producing the xanthate from the starting materials is described briefly below.

A desired amount of an alkali metal hydroxide such as potassium hydroxide or an alkali metal such as potassium metal is added into a solution of desired amount of alcohol in an aliphatic hydrocarbon or aromatic hydrocarbon or into an alcohol in an excess amount to the alkali. The mixture is allowed to react until the alkali metal or the alkali metal hydroxide is completely dissolved. Subsequently, a predetermined amount of carbon disulfide is gradually added, and the mixture is allowed to react for about 30 minutes. Thereto, a predetermined amount of p-xylylene dichloride is added and is allowed to react for about 3 hours. After the reaction, the reacted solution is concentrated and evaporated to dryness. The dried matter is dissolved in a non-halogen type solvent such as tetrahydrofuran or benzene and is then filtered. The filtrate is washed with water and concentrated to give a crude product.

In production of the polymer, the obtained crude product is preferably purified by recrystallization from a benzene/methanol type solvent. If the product is liquid, it is preferably purified by separation by column chromatography or the like means since the product is not distillable.

The radical-polymerizable monomers which can be polymerized by the xanthate of the present invention includes diene monomers such as chloroprene, 2,3-dichlorobutadiene, butadiene, isoprene and 1-chlorobutadiene; acrylic ester monomers such as methyl acrylate, ethyl acrylate, n-propyl acrylate, isopropyl acrylate, cyclohexyl acrylate, β-hydroxyethyl acrylate, β-hydroxypropyl acrylate, glycidyl acrylate, benzyl acrylate, phenoxyethyl acrylate, 2-hydroxy-3-phenoxypropyl acrylate, tetrahydrofurfuryl acrylate, isobornyl acrylate, dicyclopentenyloxyethyl acrylate, n-butyl acrylate, sec-butyl acrylate, t-butyl acrylate, methyl methacrylate, cyanoethyl acrylate and cyanobutyl acrylate; vinyl monomers such as vinyl acetate, vinyl ethyl ether, vinylidene chloride and vinyl chloride; and acrylic acid monomers such as acrylic acid and methacrylic acid.

The monomers are not limited at all to the above ones, but a great variety of monomers are polymerizable by the present invention. Theoretically, any radical-polymerizable monomers can be polymerized according to the present invention.

Further, combination of two or more of the above-mentioned monomers or other radical-polymerizable monomers can be copolymerized into random copolymers.

A procedure of the polymerization is described below. A photopolymerization apparatus is preferably

employed for the polymerization. As the light source, either a low-pressure mercury lamp or a high-pressure mercury lamp may suitably be employed, although the two lamps are different in wavelength distribution.

In production of a low-molecular-weight polymer, a polymerization solvent is not required to be employed, a bulk polymerization process being practicable. However, the initiator needs to be soluble in the monomer in the bulk polymerization.

The polymerization solvent, when it is employed, is preferably the one having small chain transfer constant such as benzene, toluene and xylene.

In the polymerization in a solvent, the concentration of the monomer is preferably not more than 30% by weight. The polymerization temperature is preferably in the range of from 0°C to 90°C, more preferably from 0°C to 50°C but is not specially limited thereto. The degree of polymerization is readily controlled by changing the charge ratio of the initiator to the monomer to prepare a polymer having a desired degree of polymerization. The polymerization is normally conducted from 10 to 30 hours, which is suitably decided depending on the monomer to be polymerized.

The polymerization is terminated by stopping the light irradiation and adding a conventional polymerization inhibitor such as BHT, and, if necessary, cooling the reaction solution. The resulting polymer is isolated by pouring the reaction solution into a large amount of a poor solvent of the polymer such as methanol and hexane, according to a conventional method. The initiator is consumed completely in principle, so that the amount of the unreacted initiator remaining in the polymer is extremely small. If the unreacted initiator is desired to be eliminated completely, the isolated polymer is preferably purified by repeating reprecipitation in such a manner that the polymer is dissolved in a solvent like tetrahydrofuran and is precipitated by adding the solution into e.g. acetone.

The present invention provides a novel compound useful as a photopolymerization initiator, and a polymerization process employing the compound. The polymerization process gives a polymer of chloroprene or other monomers colored little, and allows control of the molecular weight of various polymers, by use of the compound, over a wide range, which has not been attained in the prior arts.

The alkylxanthates and the resulting polymers were analyzed with the measuring apparatuses and under the conditions shown below.

(1) GPC

| | |
|---|---|
| Apparatus: | TSK CP-8000, made by Tosoh Corporation |
| Column: | G4000Hx, and G2000HXL |
| Detector: | UV-8000 |
| Solvent: | THF |
| Flow rate: | 1.0 ml/min |
| Pressure: | 62 $kg/cm^2$ |
| Temperature: | 25°C |

(2) $^1H$-NMR

| | |
|---|---|
| Apparatus: | NMR spectrometer EM-360 made by Varian Co. |
| Solvent: | deuterated chloroform |
| Internal standard: | TMS |
| Measuring temperature: | 25°C |
| Determination of terminal groups of chloroprene polymer: | The polymerization degree was calculated from integration ratio of H at the double bond portion of chloroprene to the integration ratio of H of the initiator residue (methylene or methyl in alkoxy group). |

The present invention is described in more detail by referring to examples without limiting the invention in any way. In the Examples below, the photopolymerization was conducted by employing a high-pressure mercury lamp UM452 (450 w), and a lightening device UM-453B-A made by Ushio Denki K.K.

EXAMPLE 1

Synthesis of Xylylene bis(ethylxanthate)

11.4 g of potassium hydroxide was put into 100 ml of ethanol, and stirred for 30 minutes to prepare a potassium hydroxide solution in ethanol. Thereto 13.1 g of carbon disulfide was added, and was allowed to react for 2 hours with stirring. The reaction solution was concentrated by an evaporator, and then poured into a large amount of ether. The precipitate formed was collected by filtration. The collected matter was recrystallized from ethanol to give potassium ethylxanthate. Subsequently, 15 g of the resulting xanthate was dissolved again in ethanol, and thereto 6.8 g of p-xylylene dichloride was added. The mixture was allowed to react for 4 hours with stirring. The reaction solution was filtered to eliminate potassium chloride formed as the reaction by-product. The solvent was evaporated off, and the evaporation residue was dissolved in benzene and filtered again. The solvent was evaporated off again, and the evaporation residue was recrystallized from a benzene-alcohol mixed solvent to give 12 g of a white crystalline matter. The resulting compound was estimated to be p-xylylene bis(ethylxanthate) having the structure shown below from the measurement by $^{1}$H-NMR. The NMR data are shown below.

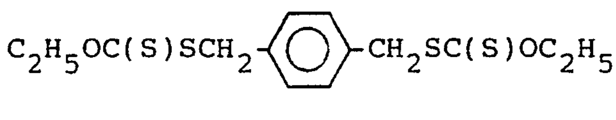

NMR Data:
$^{1}$H-NMR (60MHz, $CDCl_3$)
δ1.30 (triplet, 6H, $-O-CH_2CH_3$)
δ4.27 (singlet, 4H, $-O_6H_4-CH_2-SC(S)-$)
δ4.630 (quartet, 4H, $-O-CH_2CH_3$)
δ7.18 (singlet, 4H, aromatic H, $-C_6H_4-CH_2-$)

EXAMPLE 2

Synthesis of Xylylene bis(n-butylxanthate)

3.95 g of potassium metal was reacted with 100 ml of n-butanol for 1 hour with stirring to prepare an alcoholate solution. Thereto 13.1 g of carbon disulfide was added, and was allowed to react for 2 hours with stirring. The reaction solution was concentrated by an evaporator, and then poured into a large amount of ether. The precipitate formed was collected by filtration. The collected matter was recrystallized from ethanol to give potassium n-butylxanthate. Subsequently, 15 g of the resulting xanthate was dissolved again in ethanol, and thereto 7.6 g of xylylene dichloride was added. The mixture was allowed to react for 4 hours with stirring. The reaction solution was filtered to eliminate potassium chloride formed as the reaction by-product. The solvent was evaporated off, and the evaporation residue was dissolved in benzene and filtered again. The solvent was evaporated off again, and the evaporation residue was recrystallized from a benzene-alcohol mixed solvent to give 15 g of a white crystalline matter. The resulting compound was estimated to be p-xylylene bis(n-butylxanthate) having the structure shown below from the measurement by $^{1}$H-NMR. The NMR data are shown below.

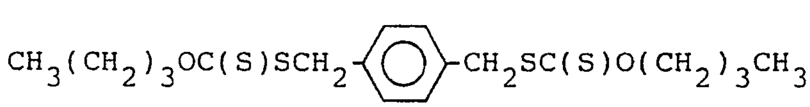

NMR Data:
$^{1}$H-NMR (60MHz, $CDCl_3$)
δ0.80-1.0 (6H, $-O-CH_2(CH_2)_2CH_3$)
δ1.0-2.0 (broad, 8H, $-O-CH_2(CH_2)_2CH_3$)
δ4.27 (singlet, 4H, $-C_6H_4-CH_2-S-C(S)-$)
δ4.53 (quartet, 4H, $-O-CH_2(CH_2)_2CH_3$)
δ7.18 (singlet, 4H, aromatic Hs, $-C_6H_4-CH_2-$)

EXAMPLE 3

Synthesis of Xylylene bis(methylxanthate)

3.95 g of sodium metal was reacted with 100 ml of methanol for 1 hour with stirring to prepare an alcoholate solution. Thereto 13.11 g of carbon disulfide was added, and was allowed to react for 2 hours with stirring. The reaction solution was concentrated by an evaporator, and then poured into a large amount of ether. The precipitate formed was collected by filtration. The collected matter was recrystallized from ethanol to give sodium methylxanthate. Subsequently, 16 g of the resulting xanthate was dissolved again in methanol, and thereto 10.8 of of xylylene dichloride was added. The mixture was allowed to react for 4 hours with stirring. The reaction solution was filtered to eliminate sodium chloride formed as the reaction by-product. The solvent was evaporated off, and the evaporation residue was dissolved in benzene and filtered again. The solvent was evaporated off again, and the evaporation residue was recrystallized from a benzene-alcohol mixed solvent to give 35 g of a pale yellow crystalline matter. The resulting compound was estimated to be p-xylylene bis(methylxanthate) having the structure shown below from the measurement by $^1$H-NMR. The NMR data are shown below.

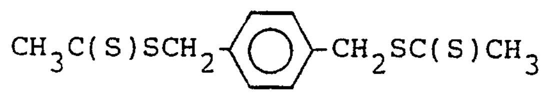

NMR Data:
$^1$H-NMR (60MHz, $CDCl_3$)
δ4.15 (singlet, 4H, $-C_6H_4-CH_2-S-C(S)-$)
δ4.33 (singlet, 6H, $-O-CH_3$)
δ7.22 (singlet, 4H, aromatic Hs, $-C_6H_4-CH_2-$)

EXAMPLE 4

Synthesis of Xylylene bis(n-propylxanthate)

3.95 g of potassium metal was reacted with 100 ml of n-propanol for 1 hour with stirring to prepare an alcoholate solution. Thereto 13.11 g of carbon disulfide was added, and was allowed to react for 2 hours with stirring. The reaction solution was concentrated by an evaporator, and then poured into a large amount of ether. The precipitate formed was collected by filtration. The collected matter was recrystallized from ethanol to give potassium propylxanthate. Subsequently, 15 g of the resulting xanthate was dissolved again in n-propanol, and thereto 9.30 g of xylylene dichloride was added. The mixture was allowed to react for 4 hours with stirring. The reaction solution was filtered to eliminate potassium chloride formed as the reaction by-product. The solvent was evaporated off, and the evaporation residue was dissolved in benzene and filtered again. The resulting filtrate was recrystallized from a benzene-alcohol mixed solvent to give 33.8 g of a white crystalline matter. The resulting compound was estimated to be p-xylylene bis(n-propylxanthate) having the structure shown below from the measurement by $^1$H-NMR. The NMR data are shown below.

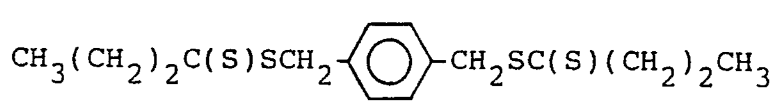

NMR Data:
$^1$H-NMR (60MHz, $CDCl_3$)
δ0.95 (triplet, 6H, $-O-CH_2CH_2CH_3$)
δ1.3-2.1 (broad, 8H, $-O-CH_2CH_2CH_3$)
δ4.30 (singlet, 4H, $-C_6H_4-CH_2-S-C(S)-$)
δ4.50 (triplet, 4H, $-O-CH_2CH_2CH_3$)
δ7.25 (singlet, 4H, aromatic Hs, $-C_6H_4-CH_2-$)

EXAMPLE 5

0.7196 g of xylylene bis(methylxanthate), 50.0 ml of benzene, and 9.971 g of chloroprene were placed

in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 30 °C for 17 hours without agitation. Subsequently, the ampul was opened, and the content of the ampul was poured into a large amount of methanol to isolate the polymer. The resulting polymer was a pale yellow viscous liquid. The number-average molecular weight (Mn) of the polymer was 2,300 according to GPC measurement. The GPC elution curve is shown in Fig. 1. The molecular weight derived from end group determination by $^1H$-NMR was 2,740. These molecular weights are approximate to the theoretical molecular weight of 2,560. From HPLC analysis (high-speed liquid chromatography), the initiator was found to be completely consumed. Above these results, the resulting polymer is estimated to have the structure below.

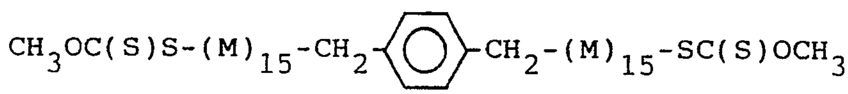

where M represents a chloroprene residue.
NMR Data:
$^1H$-NMR (60MHz, $CDCl_3$)
δ2.33 (multiplet, $-CH_2-CH=CCl-CH_2-$)
δ4.05 (singlet, $-C_6H_4-p-CH_2-$)
δ4.15, 4.30 (singlet, singlet, $-C(S)O-CH_3$)
δ5.45 (broad, $-CH=CCl-$)
δ7.05 (broad, $-O_6H_4-CH_2-$)

EXAMPLE 6

0.7828 g of xylylene bis(ethylxanthate), 50.0 ml of benzene, and 9.997 g of chloroprene were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 30 °C for 17 hours without agitation. Subsequently, the ampul was opened, and the content of the ampul was poured into a large amount of methanol to isolate the polymer. The resulting polymer was a pale yellow viscous liquid (yield: 67%). The number-average molecular weight (Mn) of the polymer was 1,920 according to GPC measurement. The GPC elution curve is shown in Fig. 1. The molecular weight derived from end group determination by $^1H$-NMR was 3,370. These molecular weights are approximate to the theoretical molecular weight of 2,970. Fig. 2 shows the NMR pattern of the polymer. From HPLC analysis, the initiator was found to be completely consumed.

From the above, the resulting polymer is estimated to have the structure below.

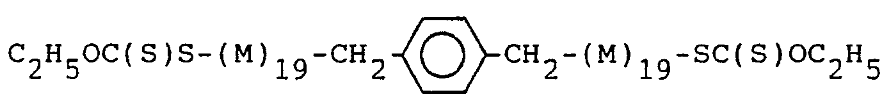

where M represents a chloroprene residue.
NMR Data:
$^1H$-NMR (60MHz, $CDCl_3$)
δ1.0-1.4 (broad, $-CS-O-CH_2CH_2CH_3$)
δ2.30 (multiplet, $-CH_2-CH=CCl-CH_2-$)
δ4.30 (singlet, $-C_6H_4-p-CH_2-$)
δ4.50 (triplet, $-O-CH_2CH_2CH_3$)
δ5.45 (broad, $-CH=CCl-$)
δ7.0-7.2 (broad, $-C_6H_4-CH_2-$)

EXAMPLE 7

0.8463 g of xylylene bis(n-propylxanthate), 50.0 ml of benzene, and 10.1657 g of chloroprene were

placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 30 °C for 17 hours without agitation. Subsequently, the ampul was opened, and the content of the ampul was poured into a large amount of methanol to isolate the polymer. The resulting polymer was a pale yellow viscous liquid (yield: 79%). The number-average molecular weight (Mn) of the polymer was 2,240 according to GPC measurement. The molecular weight derived from terminal group determination by $^1$H-NMR was 3,460. These molecular weights are approximate to the theoretical molecular weight of 3,100. From HPLC analysis, the initiator was found to be completely consumed.

From the above, the resulting polymer is estimated to have the structure below.

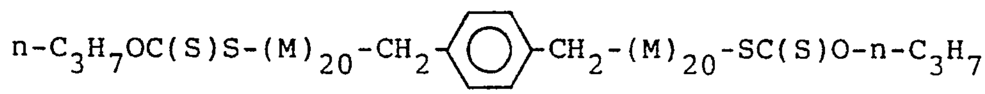

where M represents a chloroprene residue.

NMR Data:

$^1$H-NMR (60MHz, $CDCl_3$)

δ0.8-1.2 (broad, $-CS-O-CH_2CH_2CH_3$)
δ2.33 (multiplet, $-CH_2-CH=CCl-CH_2-$)
δ4.30 (singlet, $-O_6H_4$-p-$CH_2$-)
δ4.60 (triplet, $-O-CH_2CH_2CH_3$)
δ5.45 (broad, $-CH=CCl-$)
δ7.0-7.2 (broad, $-C_6H_4-CH_2-$)

EXAMPLE 8

1.3039 g of xylylene bis(methylxanthate), 350.0 ml of benzene, and 100.0 g of chloroprene were placed in a 500-ml Pyrex glass ampul. Then the ampul was deaerated sufficiently, and was melt-sealed. The polymerization was allowed to proceed by irradiating the ampul with ultraviolet light from a high pressure mercury lamp at a distance of 100 mm in a thermostated water bath kept at 15 °C for 20 hours without agitation. Subsequently, the ampul was opened, and the content of the ampul was poured into a large amount of methanol to isolate the polymer. The resulting polymer was a pale yellow viscous liquid (yield: 60%). The number-average molecular weight (Mn) of the polymer was 11,000 according to GPC measurement. This molecular weight is approximate to the theoretical molecular weight of 13,500.

From the above, the resulting polymer is estimated to have the structure below.

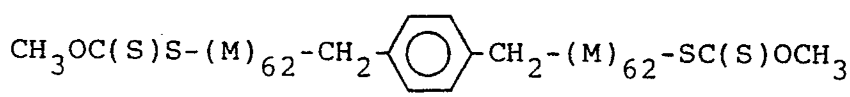

where M represents a chloroprene residue.

NMR Data:

$^1$H-NMR (60MHz, $CDCl_3$)

δ2.33 (multiplet, $-CH_2-CH=CCl-CH_2-$)
δ4.15, 4.30 (doublet, $-C(S)O-CH_3$)
δ4.05 (singlet, $-C_6H_4$-p-$CH_2$-)
δ5.43 (broad, $-CH=CCl-$)
δ7.07-7.2 (broad, $-C_6H_4-CH_2-$)

EXAMPLE 9

0.3412 g of p-xylylene bis(ethylxanthate) prepared in Example 1, 50.0 ml of benzene, and 10.0 g of ethyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 70 mm in a thermostated water bath kept at 20 °C for

22 hours without agitation. Subsequently, the ampul was opened, and the content of the ampul was poured into a large amount of hexane to isolate the polymer. The resulting polymer was an almost colorless, slightly pale yellow viscous liquid (yield: 72%). The GPC analysis of the polymer showed that the number-average molecular weight (Mn) of the polymer was 7,400 which is approximate to the theoretical molecular weight of 7,210.

EXAMPLE 10

0.4987 g of p-xylylene bis(methylxanthate) prepared in Example 3, 50.0 ml of benzene, and 10.0 g of n-butyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 70 mm in a thermostated water bath kept at 20 °C for 17 hours without agitation. Subsequently, the ampul was opened, and the content of the ampul was poured into a large amount of methanol to isolate the polymer. The resulting polymer was an almost colorless, slightly pale yellow viscous liquid (yield: 60%). The GPC analysis of the polymer showed that the number-average molecular weight (Mn) of the polymer was 3,680 which is approximate to the theoretical molecular weight of 3,830.

EXAMPLE 11

0.9094 g of p-xylylene bis(n-butylxanthate) prepared in Example 2, 50.0 ml of benzene, and 10.067 g of chloroprene were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 30 °C for 17 hours without agitation. Subsequently, the ampul was opened, and the content of the ampul was poured into a large amount of methanol to isolate the polymer. The resulting polymer was an almost colorless, slightly pale yellow viscous liquid (yield: 79%). From the determination of the end groups by $^1$H-NMR, this polymer had a number-average molecular weight (Mn) of 2,730, which is approximate to theoretical value of 3,100 calculated from the quantity of the starting materials.

EXAMPLE 12

0.1871 g of p-xylylene bis(n-propylxanthate) prepared in Example 4, 50.0 ml of benzene, and 10.0 g of ethyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 70 mm in a thermostated water bath kept at 20 °C for 17 hours without agitation. Subsequently, the ampul was opened, and the content of the ampul was poured into a large amount of methanol to isolate the polymer. The resulting polymer was an almost colorless, slightly pale yellow viscous liquid (yield: 80%). The GPC a nalysis of the polymer showed that the number-average molecular weight (Mn) of the polymer was 15,800 which is approximate to the theoretical molecular weight of 16,020.

COMPARATIVE EXAMPLE 1

0.9036 g of xylylene bis(diethyldithiocarbamate), 50.0 ml of benzene, and 9.663 g of chloroprene were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 30 °C for 17 hours without agitation. Subsequently, the ampul was opened, and the content of the ampul was poured into a large amount of methanol to isolate the polymer. The resulting polymer was a viscous liquid (yield: 59%). The GPC analysis of the polymer showed that the number-average molecular weight (Mn) of the polymer was 13,300 which is more than six times that of the theoretical value of 2,210 calculated from the quantities of the starting materials. The elution curve of the GPC is shown in Fig. 1. The resulting polymer was colored brown remarkably. Approximately 40% of the polymerization initiator remained unreacted.

COMPARATIVE EXAMPLE 2

0.2089 g of xylylene bis(diethyldithiocarbamate), 50.0 ml of benzene, and 10.0 g of chloroprene were

placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 15° C for 22 hours without agitation. Subsequently, the ampul was opened, and the content of the ampul was poured into a large amount of methanol to isolate the polymer. The resulting polymer was a viscous liquid (yield: 54%). The GPC analysis of the polymer showed that the number-average molecular weight (Mn) of the polymer was 2,460 which is greatly different from the theoretical value of 10,500 calculated from the quantities of the starting materials. The control of the molecular weight was difficult. The elution curve of the GPC is shown in Fig. 1. The resulting polymer was colored dark brown.

COMPARATIVE EXAMPLE 3

0.2224 g of xylylene bis(diethyldithiocarbamate), 50.0 ml of benzene, and 13.86 g of ethyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 15° C for 20 hours with agitation. Subsequently, the ampul was opened, and the content of the ampul was poured into a large amount of hexane to isolate the polymer (yield: 97%). The GPC analysis of the polymer showed that the number-average molecular weight (Mn) of the polymer was 16,700 which is greatly different from the theoretical value of 24,100 calculated from the quantities of the starting materials. The control of the molecular weight was difficult. The resulting polymer was colored pale yellow.

COMPARATIVE EXAMPLE 4

0.2156 g of xylylene bis(diethyldithiocarbamate), 50.0 ml of benzene, and 13.4 g of n-butyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 15° C for 20 hours with agitation. Subsequently, the ampul was opened, and the content of the ampul was poured into a large amount of hexane to isolate the polymer. The resulting polymer was a viscous liquid (yield: 89%). The GPC analysis of the polymer showed that the number-average molecular weight (Mn) of the polymer was 29,900 which is greatly different from the theoretical value of 22,200 calculated from the quantities of the starting materials. The control of the molecular weight was difficult. The resulting polymer was colored pale yellow.

COMPARATIVE EXAMPLE 5

0.642 g of xylylene bis(diethyldithiocarbamate), 50.0 ml of benzene, and 16.03 g of methyl acrylate were placed in a 100-ml Pyrex glass ampul. Then the ampul was deaerated sufficiently, and was melt-sealed. The polymerization was allowed to proceed by irradiating the ampul with ultraviolet light from a high pressure mercury lamp at a distance of 80 mm in a thermostated water bath kept at 15° C for 20 hours with agitation. Subsequently, the ampul was opened, and the content of the ampul was poured into a large amount of hexane to isolate the polymer. The resulting polymer was a viscous liquid (yield: 84%). The GPC analysis of the polymer showed that the number-average molecular weight (Mn) of the polymer was 16,300 which is greatly different from the theoretical value of 21,060 calculated from the quantities of the starting materials. The control of the molecular weight was difficult.

## Claims

1. An alkylxanthate compound represented by the general formula (I):

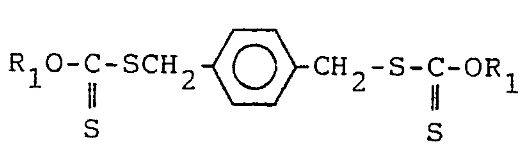

(I)

where $R_1$ is an alkyl group of 1 to 18 carbons.

**2.** A process for producing the compound of the formula (I):

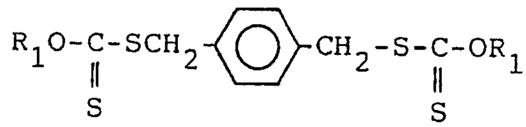

(I)

where $R_1$ is an alkyl group of 1 to 18 carbons, comprising reacting one mole of an aliphatic alcohol represented by the general formula (II)

$R_2OH$ (II)

(where $R_2$ is an alkyl group of 1 to 18 carbons) with one mole of carbon disulfide, one mole of alkali metal hydroxide or alkali metal, and 0.5 mole of p-xylylene dichloride.

**3.** A photopolymerization initiator, comprising the alkylxanthate represented by the general formula (I):

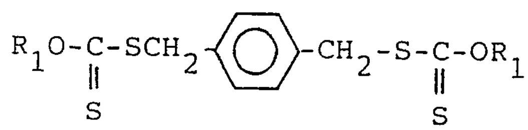

(I)

where $R_1$ is an alkyl group of 1 to 18 carbons.

**4.** A process for producing a polymer having a xanthate end group at both ends, comprising irradiating a mixture of an alkylxanthate represented by the general formula (I):

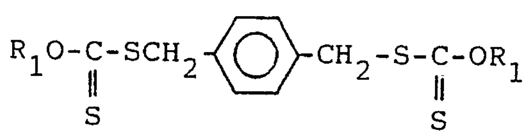

(I)

where $R_1$ is an alkyl group of 1 to 18 carbons, and a radical-polymerizable monomer with ultraviolet light to cause polymerization.

**5.** A chloroprene polymer represented by the general formula (III):

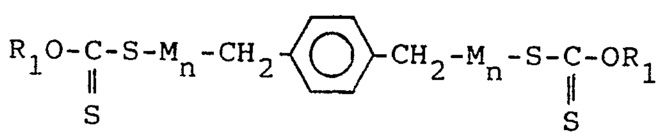

(III)

where $R_1$ is an alkyl group of 1 to 18 carbons; M is a chloroprene radical; n denotes the polymerization degree, and having a number-average molecular weight of from 1,000 to 15,000.

FIG. 1

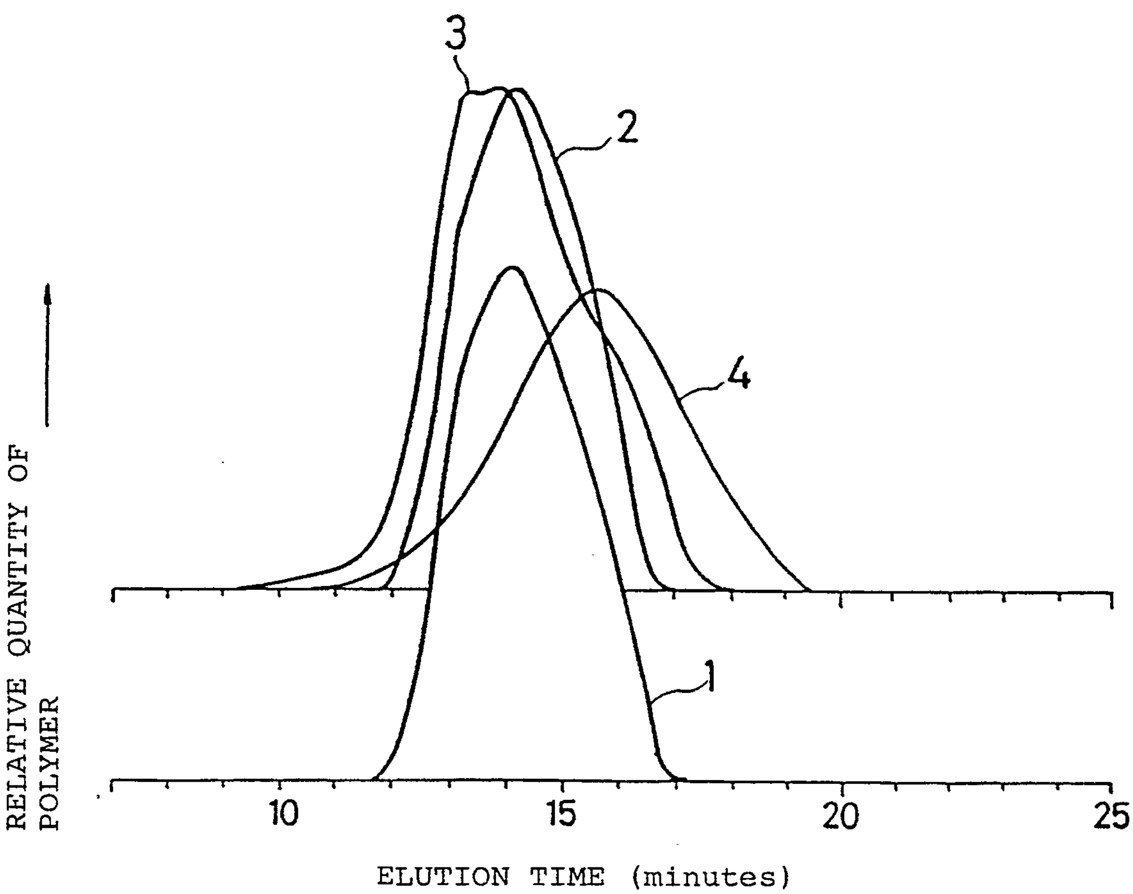
RELATIVE QUANTITY OF POLYMER
1
2
3
4
10
15
20
25
ELUTION TIME (minutes)

FIG. 2

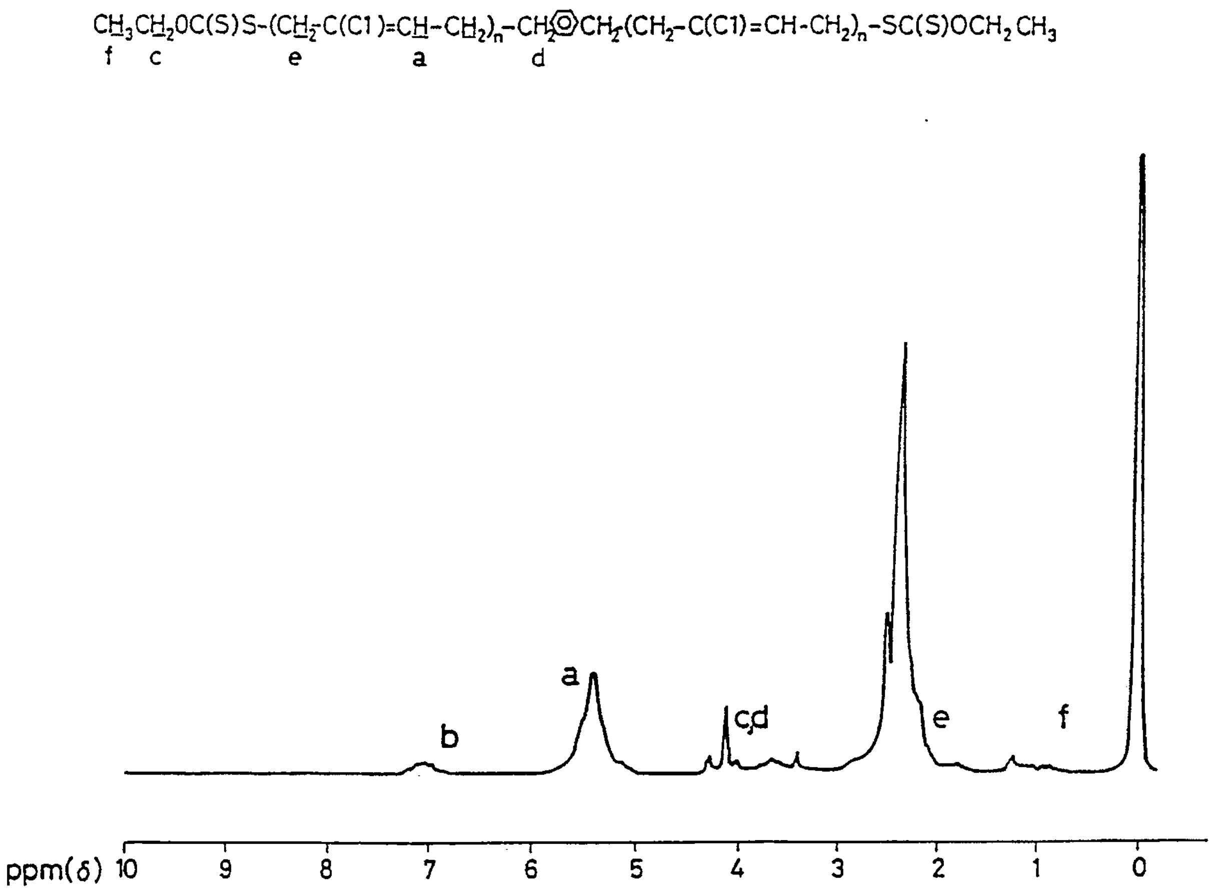

European Patent Office

# EUROPEAN SEARCH REPORT

Application Number

EP 91104876.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 68, No. 18, April 29, 1968, Columbus, Ohio, USA M. Okawara et al. "Synthesis and photochemical reactions of polymers containing dithiocarbamate and xanthate groups and their model compounds" page 7609, columns 1,2, abstract-No. 78 731n & Bull. Tokyo Inst. Techn. 78,1-16 (1966) | 1,3 | C 07 C 329/16<br>C 08 F 2/48<br>C 08 F 36/18<br>C 08 F 136/18 |
| A | DE - A1 - 3 146 772 (MERZ & CO) * Claims 1,2 * | 1,2 | |
| A | US - A - 4 777 190 (I. SASAKI et al.) * Claims * | 3,4 | TECHNICAL FIELDS SEARCHED (Int. Cl.5) |
| A | DE - A1 - 3 535 248 (BAYER AG) * Claims * | 4,5 | C 07 C 329/00<br>C 08 F 2/00<br>C 08 F 36/00<br>C 08 F 136/00 |

The present search report has been drawn up for all claims

| Place of search | Date of completion of the search | Examiner |
|---|---|---|
| VIENNA | 18-07-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document